# EUROPÄISCHE PATENTANMELDUNG

(11) **EP 0 689 840 A1**
(43) Veröffentlichungstag der Anmeldung: **03.01.1996**
(21) Anmeldenummer: 95810428.3
(22) Anmeldetag: 26.06.1995
(51) Int. Cl.: A61K 31/71, A61K 9/16, A61K 9/50

(54) **Neues orales Erythromycinbase enthaltendes Arzneimittel**

(30) Priorität: 28.06.1994 CH 2048/94
(71) Anmelder: SPIRIG AG PHARMAZEUTISCHE PRÄPARATE, CH-4622 Egerkingen (CH)
(72) Erfinder: Birrenbach, Gerd, CH-4616 Kappel (CH); Juch, Rolf Dieter, CH-4612 Wangen b. Olten (CH)
(74) Vertreter: Braun, André, jr.

(57) **Zusammenfassung**

Erythromycinbase enthaltende Grundpellets, denen zwecks besserer Freisetzung des Wirkstoffs im Darmtrakt eine geringe Menge eines schwach sauren Salzes, wie Kaliumdihydrogenphosphat, und gegebenenfalls ein Füllstoff zugesetzt ist, werden mit einem Coating versehen, das ein magensaftresistentes Polymer, wie Poly(methacrylsäure, -ethylacrylat), ein Trennmittel, wie Talcum, und einen Weichmacher, wie Diethylphthalat, enthält.

## Beschreibung

Die Erfindung betrifft ein neues orales Erythromycin enthaltendes Arzneimittel, wie es in Patentanspruch 1 beschrieben ist.

Das Makrolid-Antibiotikum Erythromycin hat sich seit langem als bakteriostatisch oder bakterizid wirkendes Arzneimittel bewährt. Üblicherweise wird es in Form von Estern, z.B. als Ethyl-succinat, Stearat, Estolat, Glucoheptonat oder Lactobionat, verwendet, was das Volumen des Fertig-Arzneimittels wegen des Esterrests stark vergrössert.

Weil sich hingegen die Erythromycinbase wohl leicht im sauren Milieu, aber nur schwer im basischen Milieu löst, ist es problematisch, im basischen Darmtrakt eine schnelle Freisetzung des Wirkstoffs ohne Zugabe grosser Mengen an Hilfsstoffen zu erreichen. Durch Zusatz von ca. 4-10 Gew. -%, bezogen auf ein ungecoatetes Grundpellet, sehr gut wasserlöslicher Verbindungen, wie PEG, Saccharose, Nicotinamid oder Adenosintriphosphat, konnte eine rasche Freisetzung des Wirkstoffs z.B. im Phosphatpuffer pH 7,4 nicht erreicht werden.

Andererseits muss die Erythromycinbase aber während der Magenpassage durch ein magensaftresistentes Coating geschützt werden, weil sie sich bei einem pH von <4,0 zersetzt. Weil magensaftresistente Zubereitungen, beispielsweise Tabletten mit einem Durchmesser von über 8 mm erfahrungsgemäss lange im Magen verweilen können ohne sich aufzulösen, muss die Erythromycinbase für eine schnelle Magenpassage vorteilhafterweise in Form von Pellets appliziert werden.

Die wässrige Verarbeitung von Erythromycinbase zu Pellets ist aber mit Komplikationen verbunden, weil der Wirkstoff ausgeprägte lipophile Eigenschaften aufweist und sich deshalb mit Wasser schlecht benetzen lässt.

Es ist nun Aufgabe der Erfindung, eine Erythromycinbase enhaltende Formulierung mit hohem Wirkstoffgehalt von >80 Gew.-%, vorzugsweise > oder gleich 90 Gew.-%, bezogen auf das ungecoatete Pelletgewicht, zu finden, die den Wirkstoff im Darmtrakt rasch freisetzt.

Eine weitere Aufgabe der Erfindung besteht darin, das Herstellungsverfahren zur Vermeidung von organischen Lösungsmitteln im Produkt und in der Umwelt, auf ausschliesslichen Einsatz von Wasser abzustellen.

Gelöst wird die Aufgabe durch die im kennzeichnenden Teil der Patentansprüche definierten Merkmale.

Überraschenderweise hat es sich gezeigt, dass die Verwendung geringer Mengen von toxikologisch und physiologisch akkzeptabeln schwach sauren Salzen die Freisetzung der Erythromycinbase im Phosphatpuffer pH 7,4 stark erhöht. So wurde z.B. durch Beimengung von ca. 1-15 Gew.-%, bezogen auf das ungecoatete Pelletgewicht, vorzugsweise 3-8 Gew.-%, besonders bevorzugt etwa 4 Gew.-%, KH₂PO₄ zur Pelletformulierung eine Auflösung der Pellets innert 15 Minuten erreicht.

Weitere geeignete schwach saure Salze sind z.B. Kaliumdihydrogencitrat, Kaliumhydrogentartrat, Kaliumhydrogenphthalat, Kaliumdihydrogenphosphat, Natriumdihydrogenphosphat oder di-Natriumhydrogencitrat, die einzeln oder als Gemisch verwendet werden können.

Um die Festigkeit der Pellets zu erhöhen, können Füllstoffe, wie z.B. Laktose, Kaolin, Dicalciumphosphat, Maltose, Maisstärke oder bevorzugt mikrokristalline Cellulose, in einer Menge von 0-20 Gew.-%, bevorzugt 3-9 Gew.-%, besonders bevorzugt etwa 6 Gew.-%, beigemengt werden.

Die erfindungsgemässen Pellets werden mit Hilfe eines Rotationsverfahrens, vorzugsweise im Rotorprozessor (z.B. Niro-Aeromatic, CH-Bubendorf) unter Besprühen der Wirkstoff- und gegebenenfalls Füllstoffmischung mit der Salzlösung und gegebenenfalls mit Wasser hergestellt, doch können gegebenenfalls auch andere herkömmliche Vorrichtungen, wie z.B. Diosna-Mischer (z.B. Dierks u. Söhne, D-Osnabrück) , unter Anpassung der Verfahrensparameter verwendet werden.

Auf diese Weise werden Pellets hoher Verdichtung und rascher Dissolution im Phosphatpuffer pH 7,4 erhalten. Auf diese Pellets wird ein magensaftresistentes Coating, welches mindestens ein magensaftresistentes Polymer, mindestens ein Trennmittel und mindestens einen Weichmacher enthält, aufgesprüht.

Das magensaftresistente Polymer wird in einer Menge von 5-40 Gew.-%, bezogen auf das Gewicht der Grundpellets ohne Coating, vorzugsweise 15-30 Gew.-%, besonders bevorzugt ca. 24 Gew.-%, das Trennmittel in einer Menge von 1-15 Gew.-%, vorzugsweise 2-10 Gew.-%, besonders bevorzugt ca. 3,75 Gew.-%, und der Weichmacher in einer Menge von 1-20 Gew.-%, bevorzugt 2-10 Gew.-%, besonders bevorzugt 2,4 Gew.-% auf das Grundpellet aufgetragen.

Als magensaftresistente Polymere eignen sich insbesondere Poly(methacrylsäure, -ethylacrylat), z.B Eudragit L30D, oder Polyvinylacetatphthalat, Celluloseacetatphthalat, Hydroxypropylmethylcelluloseacetatsuccinat oder Carboxymethylethylcellulose allein oder im Gemisch miteinander.

Als Trennmittel eignen sich z.B. Magnesiumstearat, hydriertes Rizinusöl, Dipropylglykoldipelargonat, Glyzerinmonobehenat und insbesondere Talcum allein oder im Gemisch miteinander.

Als Weichmacher eignen sich z.B. Polyethylenglykol, destillierte acetylierte Monoglyzeride, Triethylcitrat, Glyzerintriacetat, Acetyltriethylcitrat oder bevorzugt Diethylphthalat allein oder im Gemisch miteinander.

Die erfindungsgemässen Pellets werden vorzugsweise in einem Wirbelschichtgranulator mit einer wässrigen Dispersion, die aus einem Gemisch des magensaftresistenten Polymers, des Trennmittels und des Weichmachers besteht, besprüht und anschliessend getrocknet.

Die Pellets eignen sich zum Abfüllen in Hartgelatine-Kapseln. Durch den geringen Bedarf an verwendeten Hilfsstoffen und die grosse Verdichtung der Pellets weisen sie ein Schüttvolumen von ca. 130-160 ml/100 g auf. Das ermöglicht Pellets, entsprechend einer Dosis von 300 mg Erythromycinbase, in einer Gelatinekapsel der Grösse 0 zu füllen, währenddem üblicherweise eine Dosis von nur 250 mg/Kapsel üblich ist.

In den folgenden Beispielen wird die Erfindung näher erläutert.

### Beispiel 1

Es werden Grundpellets folgender Zusammensetzung hergestellt:

| | |
|---|---|
| Erythromycinbase | 90,0 g |
| Kaliumdihydrogenphosphat | 4,0 g |
| mikrokristalline Cellulose | 6,0 g |

Der Wirkstoff und der Füllstoff werden in den Rotorprozessor gefüllt. Das schwach saure Salz wird in Aqua purificata gelöst. Darauf wird die Wirkstoff/Füllstoffmischung mit der Salzlösung und anschliessend wenn nötig mit Aqua purficata besprüht, bis sich Pellets der gewünschten Grösse gebildet haben. Die Pellets werden dann durch Anheben des Innenzylinders im Prozessluftstrom getrocknet.

### Beispiel 2

Es werden Grundpellets folgender Zusammensetzung hergestellt:

| | |
|---|---|
| Erythromycinbase | 82,0 g |
| Kaliumhydrogenphthalat | 8,0 g |
| Laktose | 10,0 g |

Die Grundpellets werden wie in Beispiel 1 beschrieben hergestellt.

### Beispiel 3

Es werden Grundpellets folgender Zusammensetzung hergestellt:

| | |
|---|---|
| Erythromycinbase | 84,0 g |
| di-Natriumhydrogencitrat | 6,0 g |
| Maisstärke | 10,0 g |

Die Grundpellets werden wie in Beispiel 1 beschrieben hergestellt.

### Beispiel 4

Es werden Grundpellets folgender Zusammensetzung hergestellt:

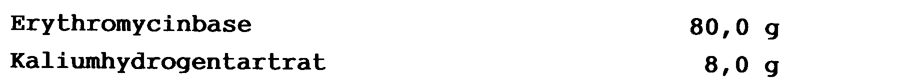

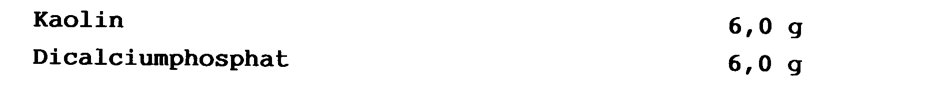

Die Grundpellets werden wie in Beispiel 1 beschrieben hergestellt.

### Beispiel 5

Auf die Grundpellets der Beispiele 1-4 wird ein Coating folgender Zusammensetzung aufgesprüht (die Angaben sind auf das Gewicht der Grundpellets berechnet):

| | |
|---|---|
| Poly(methacrylsäure, -ethylacrylat) | 23,85 % |
| Diethylphthalat | 2,40 % |
| Talcum | 3,75 % |

Vom magensaftresistenten Polymer, dem Trennmittel und dem Weichmacher wird eine wässrige Dispersion hergestellt. Die nach einem der Beispiele 1-4 hergestellten Grundpellets werden darauf im Wirbelschichtgranulator mit dieser Dispersion besprüht. Nach dem Sprühauftrag werden die gecoateten Pellets getrocknet.

### Beispiel 6

Auf die Grundpellets der Beispiele 1-4 wird ein Coating folgender Zusammensetzung aufgesprüht (die Angaben sind auf das Gewicht der Grundpellets berechnet):

| | |
|---|---|
| Celluloseacetatphthalat | 20,0 % |
| Acetyltriethylcitrat | 4,8 % |
| Magnesiumstearat | 2,2 % |

Die Grundpellets werden wie in Beispiel 5 beschrieben mit dem Coating besprüht und getrocknet.

### Beispiel 7

Auf die Grundpellets der Beispiele 1-4 wird ein Coating folgender Zusammensetzung aufgesprüht (die Angaben sind auf das Gewicht der Grundpellets berechnet):

| | |
|---|---|
| Hydroxypropylmethylcelluloseacetatsuccinat | 20,0 % |
| Triethylcitrat | 6,0 % |
| Talcum | 6,0 % |

Die Grundpellets werden wie in Beispiel 5 beschrieben mit dem Coating besprüht und getrocknet.

### Beispiel 8

Auf die Grundpellets der Beispiele 1-4 wird ein Coating folgender Zusammensetzung aufgesprüht (die Angaben sind auf das Gewicht der Grundpellets berechnet):

| | |
|---|---|
| Carboxymethylethylcellulose | 20,0 % |
| dest. acetylierte Monoglyzeride | 6,0 % |
| hydriertes Rizinusöl | 6,0 % |

Die Grundpellets werden wie in Beispiel 5 beschrieben mit dem Coating besprüht und getrocknet.

### Beispiel 9

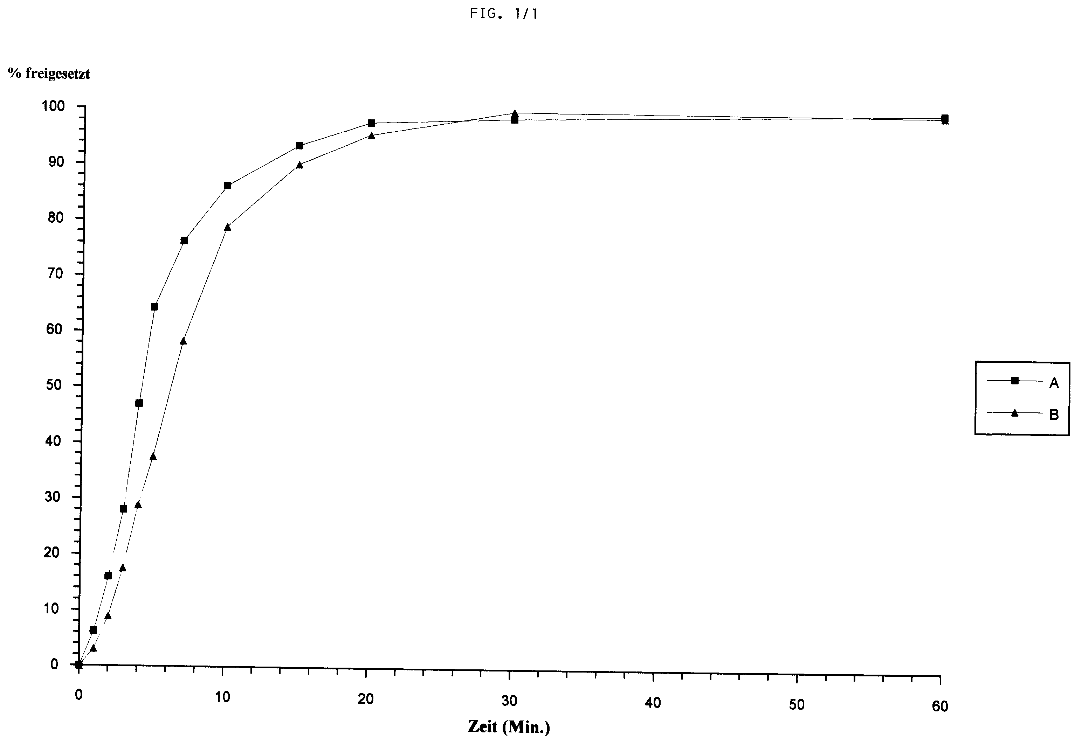

Grundpellets, hergestellt nach Beispiel 1, werden in zwei Versuchen A und B mit einem nach Beispiel 5 hergestellten Coating besprüht. Der Unterschied zwischen den Versuchen A und B besteht darin, dass das Coating bei Versuch A in zwei zeitlich voneinander gestaffelten Arbeitsgängen und bei Versuch B in einem einzigen Arbeitsgang aufgesprüht wird. Nach der Trocknung wird die in vitro Freisetzung der Erythromycinbase nach USP XXII, Phosphatpuffer pH 7,4 nach vorgängiger 2-stündiger Vorbehandlung in künstlichem Magensaft nach Ph.H. VI gemessen. Die Resultate sind in Fig. 1 aufgezeichnet.

## Patentansprüche

1. Erythromycinbase enthaltendes Arzneimittel in Pelletform, dadurch gekennzeichnet, dass die ungecoateten Grundpellets Erythromycinbase in einer Menge von >80 Gew. -%, bezogen auf das ungecoatete Pelletgewicht, mindestens ein schwach saures Salz und gegebenenfalls mindestens einen Füllstoff enthalten, und dass die Grundpellets mit einem magensaftresistenten Coating überzogen sind.

2. Arzneimittel nach Patentanspruch 1, dadurch gekennzeichnet, dass die Erythromycinbase in einer Menge von mindestens 90 Gew. -%, bezogen auf das ungecoatete Pelletgewicht, beträgt.

3. Arzneimittel nach Patentanspruch 1 oder 2, dadurch gekennzeichnet, dass das schwach saure Salz in einer Menge von ca. 1-15 Gew.-%, bezogen auf das ungecoatete Pelletgewicht, vorzugsweise 3-8 Gew.-%, besonders bevorzugt etwa 4 Gew.-%, vorliegt.

4. Arzneimittel nach einem der Patentansprüche 1-3, dadurch gekennzeichnet, dass die Grundpellets als schwach saures Salz Kaliumdihydrogencitrat, Kaliumhydrogentartrat, Kaliumhydrogenphthalat, Natriumdihydrogenphosphat oder di-Natriumhydrogencitrat, oder vorzugsweise Kaliumdihydrogenphosphat, einzeln oder als Gemisch enthalten.

5. Arzneimittel nach einem der Patentansprüche 1-4, dadurch gekennzeichnet, dass die Grundpellets als Füllstoffe Laktose, Kaolin, Dicalciumphosphat, Maltose, Maisstärke oder bevorzugt mikrokristalline Cellulose, in einer Menge von 0-20 Gew.-%, bevorzugt 3-9 Gew.-%, besonders bevorzugt etwa 6 Gew.-%, enthalten.

6. Arzneimittel nach einem der Patentansprüche 1-5, dadurch gekennzeichnet, dass das magensaftresistente Coating ein magensaftresistentes Polymer, mindestens ein Trennmittel und mindestens einen Weichmacher enthält.

7. Arzneimittel nach einem der Patentansprüche 1-6, enthaltend ein Coating aus 5-40 Gew.-%, jeweils bezogen auf das Gewicht der Grundpellets ohne Coating, vorzugsweise 15-30 Gew.-%, besonders bevorzugt ca. 24 Gew.-%, mindestens eines magensaftresistenten Polymer, 1-15 Gew.-%, vorzugsweise 2-10 Gew.-%, besonders bevorzugt ca. 3,75 Gew.-%, mindestens eines Trennmittels und 1-20 Gew.-%, bevorzugt 2-10 Gew.-%, besonders bevorzugt 2,4 Gew.-% mindestens eines Weichmachers.

8. Arzneimittel nach Patentanspruch 7, enthaltend als magensaftresistents Polymer Polyvinylacetatphthalat, Celluloseacetatphthalat, Hydroxypropylmethylcelluloseacetatsuccinat oder Carboxymethylethylcellulose oder vorzugsweise Poly(methacrylsäure, -ethylacrylat), allein oder im Gemisch miteinander.

9. Arzneimittel nach Patentanspruch 7, enthaltend als Trennmittel Magnesiumstearat, hydriertes Rizinusöl, Dipropylglykoldipelargonat, Glyzerinmonobehenat oder insbesondere Talcum allein oder im Gemisch miteinander.

10. Arzneimittel nach Patentanspruch 7, enthaltend als Weichmacher Polyethylenglykol, destillierte acetylierte Monoglyzeride, Triethylcitrat, Glyzerintriacetat, Acetyltriethylcitrat oder bevorzugt Diethylphthalat allein oder im Gemisch miteinander.
